# EUROPEAN PATENT SPECIFICATION

(11) **EP 0 388 760 B1**
(45) Date of publication and mention of the grant of the patent: **02.08.1995**
(21) Application number: 90104710.0
(22) Date of filing: 13.03.1990
(51) Int. Cl.: G11B 7/125, G11B 7/14

(54) **Laser diode and multibeam optical head using the laser diode**
Laserdiode und optischer Kopf mit mehreren Strahlen, welcher die Laserdiode verwendet
Diode laser et tête optique à rayons multiples utilisant la diode laser

(30) Priority: 14.03.1989 JP 59564/89
(43) Date of publication of application: 26.09.1990
(73) Proprietor: FUJI XEROX CO., LTD., Minato-ku Tokyo 107 (JP)
(72) Inventor: Yasukawa, Kaoru, c/o Fuji Xerox Co., Ltd., Ebina-shi, Kanagawa (JP); Ueyanagi, Kiichi, c/o Fuji Xerox Co., Ltd., Ebina-shi, Kanagawa (JP)
(74) Representative: Boeters, Hans Dietrich, Dr.

(56) References cited:
- EP-A- 0 092 420
- US-A- 4 298 974

## Description

### BACKGROUND OF THE INVENTION

The present invention relates to a laser diode and a multibeam optical head using the the laser diode as used in an optical writing and reading system which optically performs writing, reading, erasure, and so forth of information by irradiating the convergent rays from a light source onto optical writing media.

Along with the development of information processing systems in the recent years, it has been strongly desired that a high transfer data rate will be attained, in addition to the realization of a large capacity in the past, in optical writing and reading systems, so that the writing and reading of information may be accomplished thereby. Various efforts have been made towards this goal (as observed in 4p-ZD-1, 4p-ZD-2, etc. read at Extended Abstracts (The 49th Autum Meeting, 1989); The Japan Society of Applled Physics). In this regard, it has been proposed the adoption of multibeam in an optical head, which consists in using a plural number of writing and reading converging spots in an optical head, as a means of achieving a high transfer data rate just mentioned.

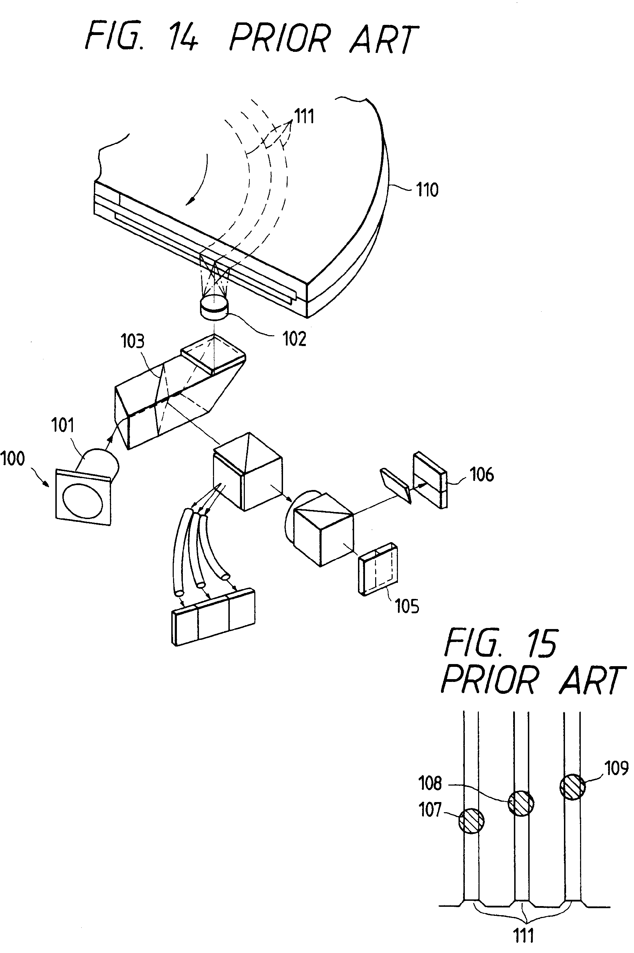

An example of this type of multibeam optical head as proposed in the past is, for example, disclosed in Japanese Patent Laid Open No. 117744/1986. As shown in Fig. 14, the optical head is so constructed as to include a laser diode array 100, in which many laser diodes independently controllable for their light emission are arranged linearly and at equal intervals to form a unified structure, a condenser lens 101, which converts the rays output from the laser diode array 100 into parallel collimated rays, an object lens 102, which converges the parallel rays mentioned above into minute spots, a polarizing beam splitter 103, which separates the incident rays and the reflected rays, and a plurality of beam splitting and detecting means 104, which detects signals by splitting the reflected rays from a plurality of beam spots, a photo-detector 105, which detects the position of one track, and a pair of focusing error detecting systems 106.

With this construction, it is made possible to perform the writing or reading of information on a plural number of tracks 111 at the same time, by irradiating a plurality of beam spots 107 through 109 (three spots in the example shown in Fig. 15) onto a plural number of tracks 111 on an optical disk 110 at the same time.

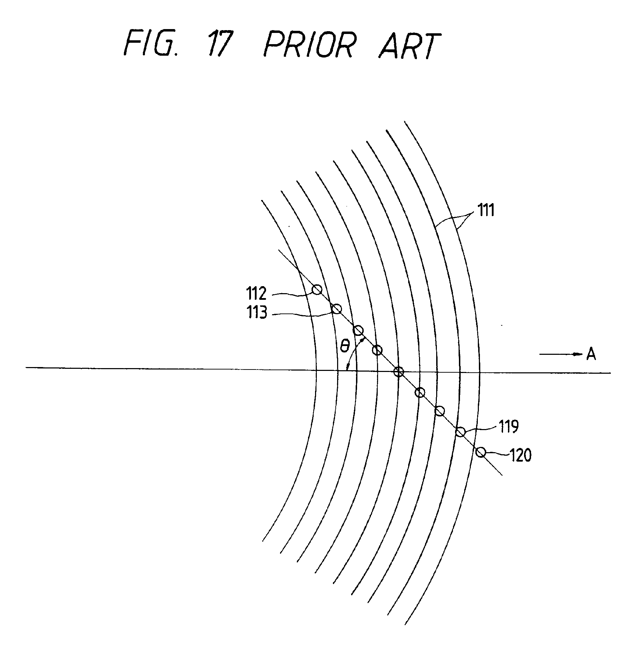

Now, in the case of the multibeam optical head mentioned above, if an attempt were made to arrange a plurality of beam spots 107 through 109 linearly at equal intervals along a line in the radial direction A of the optical disk 110, it would be necessary to set the spacing of the plurality of laser diodes arranged in the laser diode array very narrow, in consequence of such factors as the magnification of the optical system, so that it would virtually be impossible to manufacture the laser diode array 100. Therefore, as shown in Fig. 17, a plurality of beam spots 112 through 120 arranged in a straight line are set at a slant by a prescribed angle ϑ in relation to the radial direction A of the optical disk 110, so that the intervals of the plurality of laser diodes arranged in the laser diode array 100 may thereby be set at a value feasible to the actual manufacture of the array.

However, the prior art described above has such problems as those mentioned below. That is to say, the multibeam optical head described above is designed to arrange a plurality of beam spots 112 through 120 linearly at equal intervals, as shown Fig. 17. Therefore, if the many beam spots 112 through 120 were arranged in such a way as to be set at a slant at the angle ϑ in relation to the radial direction A of the optical disk 110, as shown in Fig. 17, it would not be possible to position all of the plurality of beam spots 112 through 120 over the tracks 111 corresponding to these since the tracks 111 of the optical disk 110 are formed in a circular arc having a prescribed radius of curvature, with the result that the beam spots on both ends would be positioned off the tracks 111. Consequently, it would not be possible to have all the beam spots perform the tracking of their respective tracks 111 at the same time and with accuracy even if tracking servo were applied to them, and this constitutes a problem in that the writing or reading of information could not be performed on a plurality of tracks 111 at the same time.

US-A-4,298,974 describes according to the preamble of claim 1 an optical head, which is suited for use with an optical disk for optically recording information. The optical head, which is optically coupled to the disk and is movable along a movement axis in the radial direction of the disk, comprises a laser diode array with a plurality of independently drivable lasing means which are nonuniformally spaced for generating a plurality of spaced laser beams. The resulting beam spots of the laser beams on the surface of the disk are arranged in a straight line set in a slant in relation to the radial direction of the disk.

The prior art shows the disadvantage that it is not possible to position the plurality of the generated beam spots over the tracks corresponding to these because the curvature of the tracks is not taken into consideration. Consequently, it would not be possible to have all the beam spots perform the tracking of their respective tracks at the same time and with accuracy. Therefore the reading and writing of information could not be performed on a plurality of tracks at the same time.

### SUMMARY OF THE INVENTION

Therefore, the present invention has been made in order to overcome the above-mentioned problem of the prior art. An object of this invention is to provide a laser diode capable of tracking all the laser beams on all the individual tracks and performing the writing or reading of information on a plurality of tracks at the same time and a multibeam optical head using the laser diode, which performs writing or reading or the like at the same time, using a plurality of laser beams.

That is to say, the optical information system according to the invention comprises an optical recording means having a plurality of circular tracks uniformely spaced one from another in a radial direction of said optical recording means for recording information thereon; and a multibeam optical head means optically coupled to said optical recording means and movable along a movement axis in said radial direction, said multibeam optical head means comprising: a laser diode means having a plurality of lasing means nonuniformely spaced one from another for generating a plurality of spaced laser beams, and image forming optical means, coupled to said laser diode means, for directing each of said plurality of laser beams as a beam spot onto a respective one of said plurality of tracks of the optical recording means with a common amplification, so that said beam spots are arranged in a straight line set at a slant in relation to said radial direction, wherein said plurality of lasing means is nonuniformely spaced one from another such that the spacing between adjacent ones of said beam spots corresponds to the spacing between adjacent ones of said lasing means.

As regards the manner of forming the images of the beam spots, one spot is positioned on the axis for the transit of the optical head in the radial direction of the optical writing media while the remaining spots are positioned in equal numbers on both sides of the transit axis, for the formation of images, in case the number of the beam spots is an odd number, for example.

Also, in case the number of the beam spots mentioned above is an even number, the images are formed by positioning the beam spots in equal numbers on both sides of the transit axis, for example, in relation to the axis of the transit of the optical head in the radial direction of the optical writing media.

However, the manner of image formation with the beam spots mentioned above is not limited to the image forming manners just described, but it is, of course, feasible to have the image formed with one beam spot positioned on the axis of transit for the optical head in the radial direction of the optical writing media and with the other beam spots being positioned on either one side of the axis of transit for the optical head.

Moreover, for the error detection for tracking servo, it is desirable to obtain a basis therefor, for example, from the reflected rays of the spot positioned on the axis for the transit of the optical head in the radial direction of the optical writing media, in case the number of beam spots is an odd number, but from the reflected rays of the spot nearest to the axis for the transit of the optical head in the radial direction of the optical writing media in case the number of the beam spots is an even number.

Furthermore, in order to separate the beam spot for the performance of the error detection for tracking servo out of a plurality of beam spots, a pin hole, for example, is used.

In the laser diode according to this invention, a plurality of light emitting elements are arranged in a straight line with their intervals varied, and it is therefore possible to irradiate all the laser beams emitted from a plurality of light emitting elements on the tracks of all the optical writing media by arranging a plurality of light emitting elements with a variation by the prescribed amount, depending on the curvature of the tracks, even in case it is designed to irradiate the laser beams emitted from a plurality of light emitting elements over the tracks at the same time.

Moreover, the multibeam optical head according to this invention is constructed in such a way as to be provided with a laser diode wherein a plurality of independently drivable light emitting elements are arranged at varying intervals in a straight line and with an image forming optical system which forms images by directing a plurality of beams from the laser diode mentioned above to the optical writing media in such a way as to arrange a plurality of beam spots linearly at a prescribed angle to the radial direction of the optical writing media and to vary the intervals therebetween, and the multibeam optical head is therefore capable of forming the images by focusing all of the plurality of laser beams emitted from the laser diode on the tracks on the optical writing media at the same time even in case all of the plurality of laser beams are irradiated at the same time to form the images arranged linearly at a prescribed angle to the radial direction of the optical writing media.

### BRIEF DESCRIPTION OF THE DRAWINGS

Fig. 1 is a schematic construction view illustrating one embodiment in an optical writing and reading system to which a multibeam optical head according to this invention is applied;
Fig. 2 and Fig. 3 are respectively a construction view and perspective view of the optical writing and reading system;
Fig. 4 is a circuit diagram showing the detecting section for the tracking error signal;
Fig. 5 is a perspective view showing the principle of the detection of the focusing error signal;
Fig. 6 is a circuit diagram showing the detecting section for the focusing error signal;
Figs. 7 (a), 7(b) and 7(c) are perspective views respectively showing the detecting operations for the focusing error signal;
Fig. 8 is a perspective view showing the laser diode;
Fig. 9 is a plane view showing the state of image formation with the multibeam;
Fig. 10 is an explanatory view illustrating the method of setting the intervals of the multibeams;
Fig. 11 is a plane view showing the state of image formation with this multibeam in illustration of another embodiment of this invention;
Fig. 12 is a perspective view showing the laser diode;
Fig. 13 is an explanatory view showing a method of setting the intervals of the multibeam;
Fig. 14 is a perspective view showing a conventional multibeam optical head;
Fig. 15 is a plane view illustrating the beam formed with the multibeam optical head shown in Fig. 14;

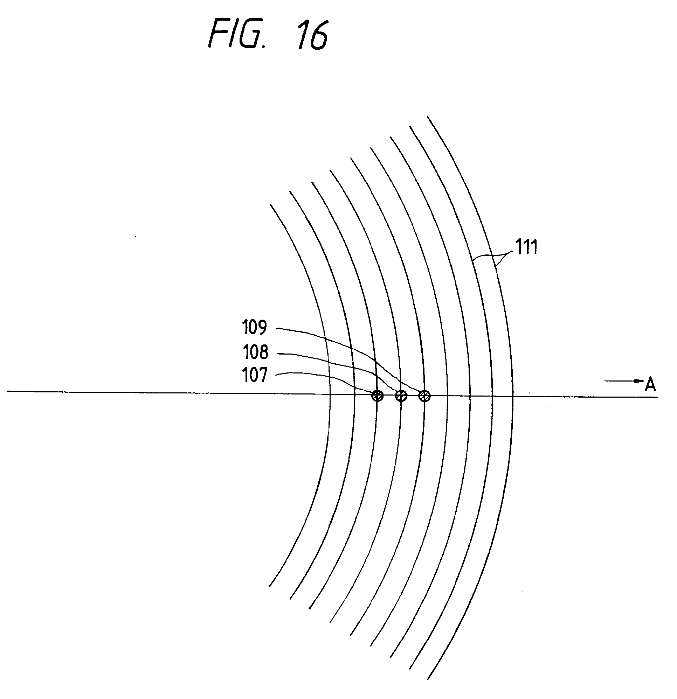

Fig. 16 is an explanatory view showing one manner of image formation with the multibeams; and
Fig. 17 is an explanatory chart showing another manner of image formation with the multibeams.

### DETAILED DESCRIPTION OF THE PREFERRED EMBODIMENTS

Preferred embodiments of the present invention will now be described with reference to the accompanying drawings.

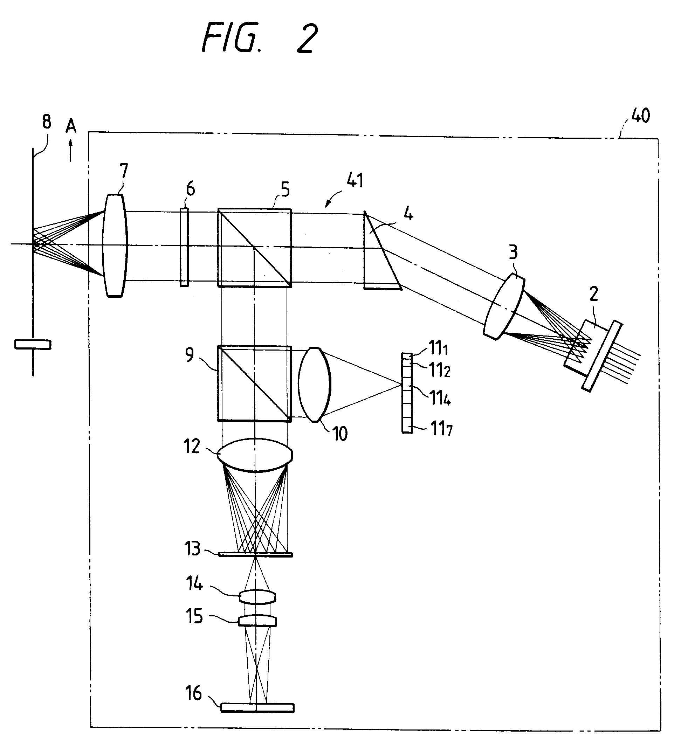

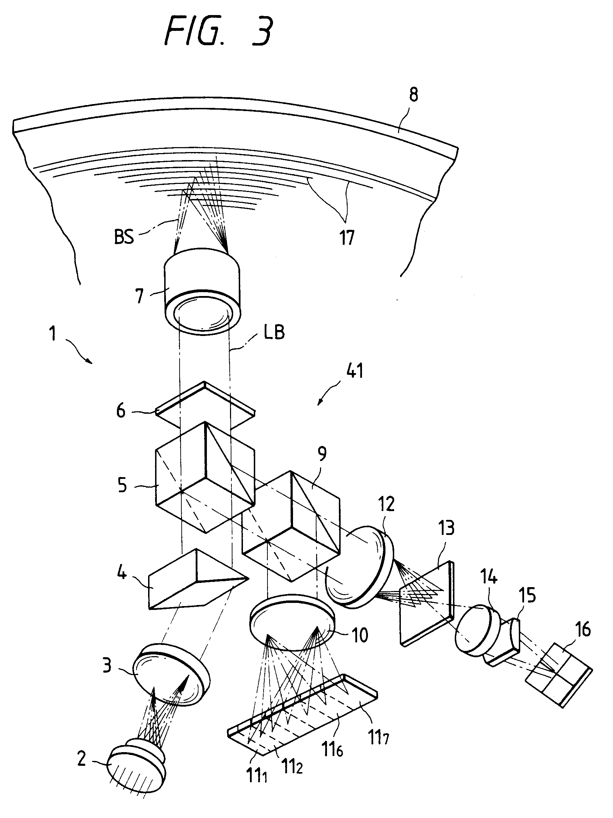

Fig. 2 and Fig. 3 illustrate an optical writing and reading system to which a laser diode and a multibeam optical head both according to the present invention are applied.

This optical writing and reading system 1 is comprised of a laser diode 2, which is composed of a plurality of independently drivable light emitting elements arranged in a straight line, a collimator lens 3, which converts into parallel rays a plurality of the laser beams LB₁ through LB₇ (seven pieces in the Figures) having an elliptical sectional shape as emitted from the laser diode 2, a beam shaping prism 4, which conditions into circular sectional shape the laser beams LB₁ through LB₇ as thus converted into parallel rays by the collimator lens 3, a polarizing beam splitter 5, which separates these shaped laser beams LB₁ through LB₇ between the rays irradiated to an optical disk 8 to be mentioned later and the rays reflected from the optical disk 8, a one-quarter wavelength plate 6, which converts the linear deflected rays and the circular deflected rays reciprocally from one into the other, an object lens 7, which converges and irradiates the laser beams LB₁ through LB₇ having passed through the one-quarter wavelength plate 6 onto the optical disk 8, the optical disk 8 as the optical writing media onto which the laser beams LB₁ through LB₇ converged by the object lens 7 are irradiated, a beam splitter 9, which further split into two parts the reflected laser beams LB₁ through LB₇ as separated by the polarizing beam splitter 5 mentioned above, a condenser lens 10, which collects one part of the laser beams LB₁ through LB₇ as split by the beam splitter 9, seven units of photodetector elements 11₁ through 11₇, which receive the part of the laser beams LB₁ through LB₇ as collected by the condenser lens 10, a condenser lens 12, which collects the other part of the laser beams LB₁ through LB₇ as split by the beam splitter 9 mentioned above, a pinhole 13, which permits the passage of only the middle laser beam LB₄ among the laser beams LB₁ through LB₇ collected by the condenser lens 12, a condenser lens 14 and a cylindrical lens 15, which detect the focusing error signal by applying the astigmatic method to the laser beam LB₄ as taken out through the pinhole 13, and a photo-detector element 16, which receives the beam processed for image formation with the condenser lens 14 and the cylindrical lens 15.

Also, the optical disk 8 mentioned above has the tracks 17 and 17 ... for their writing of information with a prescribed radius of curvature and a prescribed pitch p in the form of concentric circles, as shown in Fig. 3.

Then, in the optical writing and reading system described above, a plurality of laser beams LB₁ through LB₇ which are emitted in an elliptical shape from the laser diode 2 are converted into parallel rays with the collimator lens 3 and thereafter shaped into circular beams by means of the beam shaping prism 4, as shown in Fig. 2. After that, these laser beams LB₁ through LB₇ pass through the polarizing beam splitter 5 and the one-quarter wavelength plate 6, narrowed down with the object lens 7 and irradiated on the tracks 17, and 17 ... on the optical disk 8.

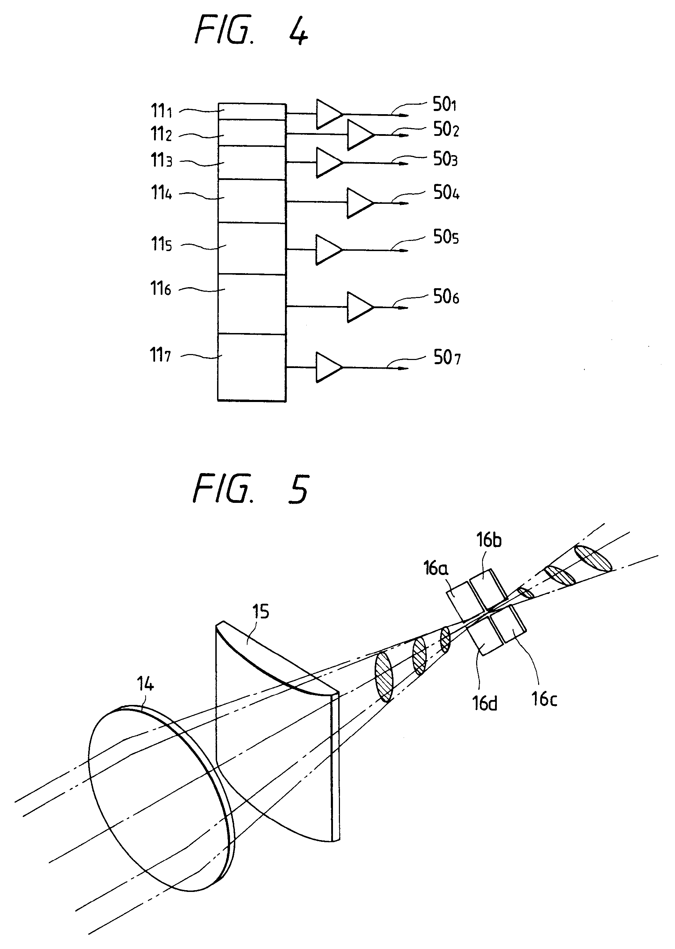

The rays reflected from the tracks 17 and 17 ... of the optical disk 8 mentioned above are fed back by the same route as what is mentioned above, and the rays are reflected by the polarizing beam splitter 5 and at the same time split by the beam splitter 9 for their transit in two directions. The laser beams LB₁ through LB₇ in one stream are applied by means of the condenser lens 10 onto the seven photo-detector elements 11₁ through 11₇ to form images, and the image information recorded in a plurality of tracks 17 and 17 ... is read at the same time by the photo-detector elements 11₁ through 11₇. As shown in Fig. 4, the individual photo-detector elements 11₁ through 11₇ are connected with differential amplifiers, and image information is obtained as output signals 50₁ through 50₇ from the individual differential amplifiers.

Now, the laser beams LB₁ through LB₇ forming the other stream as split by the beam splitter 9 mentioned above are condensed with a condenser lens 12, and thereafter only the middle laser beam LB₄ passes through the pinhole 13 and is applied to form its image onto the photo-detector element 16 by way of the condenser lens 14 and the cylindrical lens 15. Thus, a tracking error signal is obtained, and also a focusing error signal is obtained by the astigmatic process.

The tracking error signal mentioned above is obtained in the manner described below. The photo-detector element 16 is composed of mutually adjacent photo-detector elements 16a and 16b and adjacent photo receptor elements 16c and 16d, which are connected respectively with the adding amplifiers 54 and 55, and these adding amplifiers 54 and 55 are connected with the differential amplifier 56, and a tracking error signal is obtained from this differential amplifier 56.

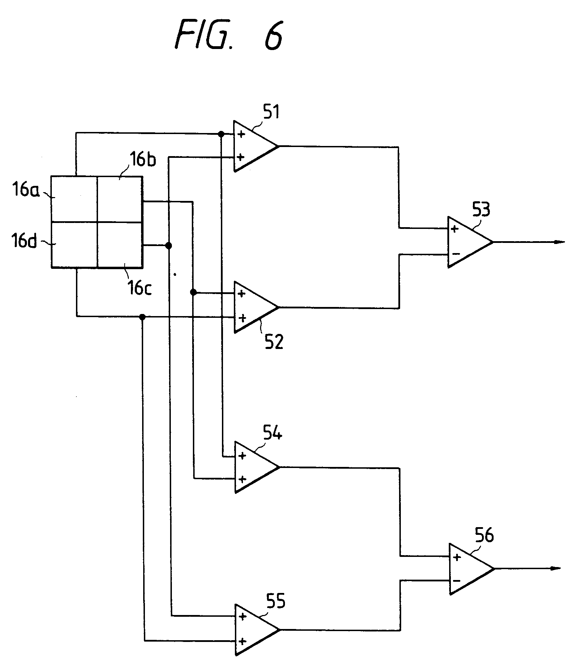

Also, the astigmatic process mentioned above, as already known, is constructed so as to obtain a focusing error signal through utilization of the feature that a circular beam is obtained in an approximately midway position in the in-focus state, as shown in Fig. 5, by varying the focus in the x-axis direction and that in the y-axis direction of the laser beam by means of the cylindrical lens 15. The quadrant photo receptor element 16 is provided with the photo-detector elements 16a and 16c and the photo-detector elements 16b and 16d positioned on diagonal lines, and these photo receptor elements are connected respectively with the adding amplifiers 51 and 52, which in its turn are connected to the differential amplifier 53, as shown in Fig. 6.

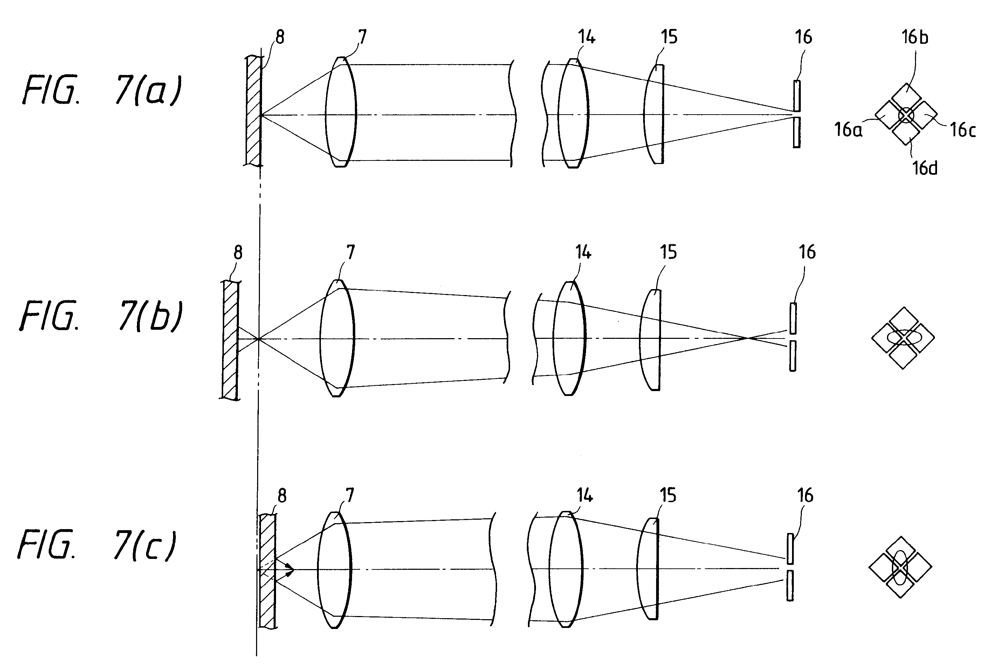

Now, the output from the differential amplifier becomes zero in the in-focus state as shown in Fig. 7 (a) whereas the output from the differential amplifier 53 becomes either + or -, as shown in Figs. 7 (b) and 7(c), and a focusing error signal is thereby obtained.

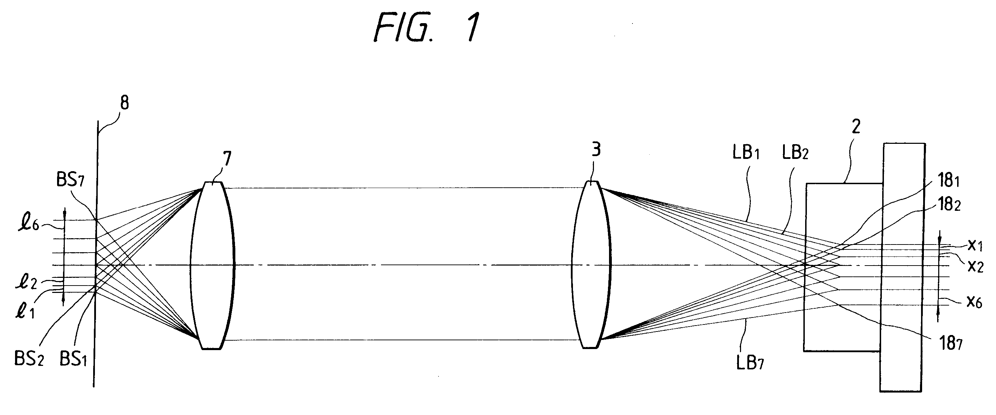

In this regard, the laser diode in accordance with the embodiment is constructed with a plurality of light emitting elements mentioned above being arranged in a straight line at varying intervals. That is to say, the laser diode 2 is provided, as shown in Fig. 1, with a plurality of laser diode elements 18₁ through 18₇ arranged in one block in a straight line in such a way that the intervals X₁ through X₆ of the individual laser diode elements increase progressively (i.e. x₁ < X₂ ... < X₆).

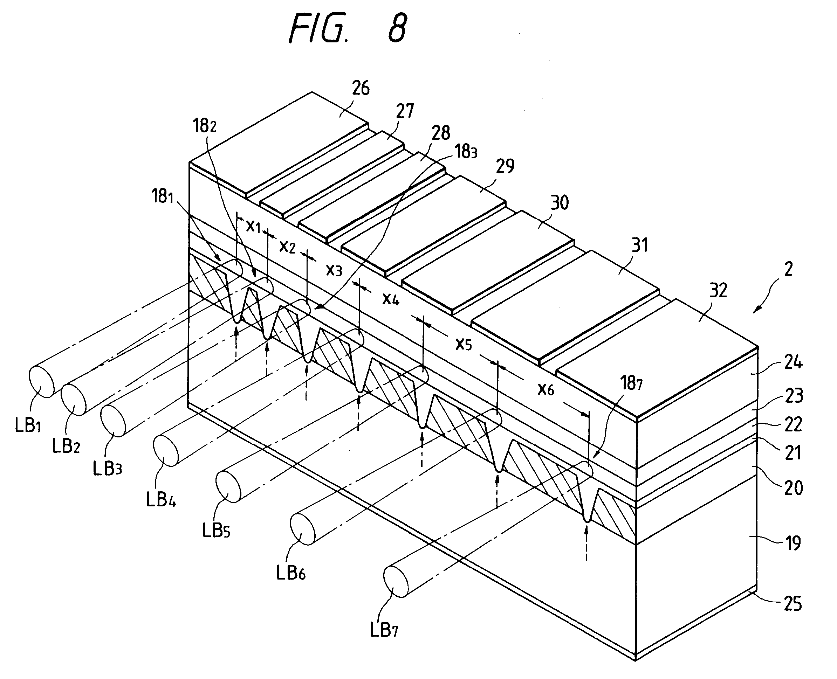

The principal part of the laser diode 2 mentioned above, as shown in Fig. 8, is composed of a p-GaAs substrate 19, an n-GaAs layer 20 formed on the substrate 19 and forming a current constricting layer, a p-Ga₁₋ₓ AlₓAs layer 21 formed on the n-GaAs layer 20 and forming a clad layer, a p-Ga_{1-y}Al_{y}As layer 22 forming an active layer formed on the p-Ga₁₋ₓAlₓAs layer 21, a p-Ga₁₋ₓAlₓAs layer 23 forming a clad layer formed on the p-Ga_{1-y}Al_{y}As layer 22, and an n-GaAs layer 24 forming a gap layer formed on the p-Ga₁₋ₓAlₓAs layer 23. A p-side electrode 25 is formed over the entire area on the back side of the p-GaAs substrate, and, on the other hand, a plurality of n-side electrodes 26 through 32 are formed on the n-GaAs layer 24 mentioned above. Also, on the n-GaAs layer 20, which forms the current constricting layer mentioned above, are provided V-shaped grooves 33 through 39 formed by the mesa-etching process or the like, and laser diode elements 18₁ through 18₇ are formed with the individual V-shaped grooves 33 through 39.

And, in an active area of the individual laser elements 18₁ through 18₇, an electric current is injected from the direction indicated by the arrow into the inside region of the current constricting layer, n-GaAs layer 20, which has a polarity reverse to that of the substrate 19, through the V-shaped grooves 33 through 39 by applying a driving voltage independently between the p-side electrode 25 and the n-side electrode 26 through 32 mentioned above. Then, laser oscillation takes place in the p- Ga_{1-y}Al_{y}As layer 22, which is the active layer, and a plurality of laser beams LB₁ through LB₇ each having an elliptical shape are obtained from the individual laser diode elements 18₁ through 18₇. These laser beams LB₁ through LB₇ are emitted at intervals equal to the intervals X₁ through X₆ of the laser diode elements 18₁ through 18₇.

Moreover, the multibeam optical head according to this embodiment is provided with a laser diode constructed as described above and an image forming optical system which directs the plural number of beams from the above-mentioned laser diode onto the optical writing media in such a way that a plurality of beam spots are arranged linearly at a prescribed angle in relation to the radial direction of the writing media and also the intervals therebetween vary.

That is to say, the multibeam optical head 40 is provided with an image forming optical system 41, which is composed mainly of the component parts of the optical writing and reading system except for the optical disk 8, in addition to the laser diode 2, and this optical head 40, as shown in Fig. 2, is so constructed as to be capable of moving along the line in the radial direction A of the optical disk 8 by the action of the driving means not shown in the Figure.

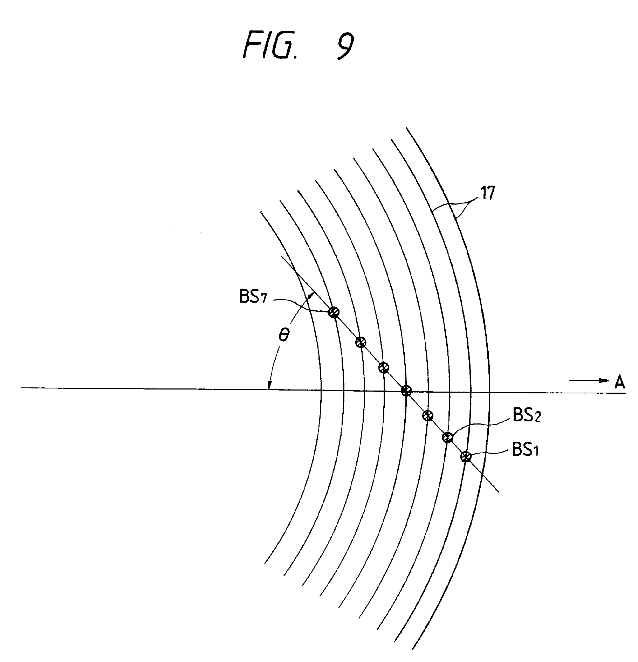

Now, a plurality of laser beams LB₁ through LB₇ emitted from the laser diode 2 mentioned above are converged with the object lens 7 through the collimator lens 3, the anamorphic prism 4, the polarizing beam splitter 5, and the one-quarter wavelength plate 6, as shown in Fig. 2, and directed in the form of a plurality of beam spots BS₁ through BS₇ onto the optical disk 8 to form images thereon in a straight line with an angle ϑ in relation to the radial direction A of the optical disk 8, as shown in Fig. 9.

Furthermore, the plural number of beam spots BS₁ through BS₇ irradiated in a straight line onto the optical disk 8 mentioned above are, as shown in Fig. 1, are designed to perform their image formation with progressive increases of their intervals (i.e., l₁ < l₂ ... < l₆), so that all the beam spots will be positioned on the tracks 17 and 17 ... on the optical disk 8. At this moment, the number of the beam spots BS₁ through BS₇ is seven, which is an odd number, and, among these beam spots BS₁ through BS₇, the middle beam spot BS₄ is so arranged as to move in the direction agreeing with the direction of movement of the optical head 40, as shown in Fig. 9.

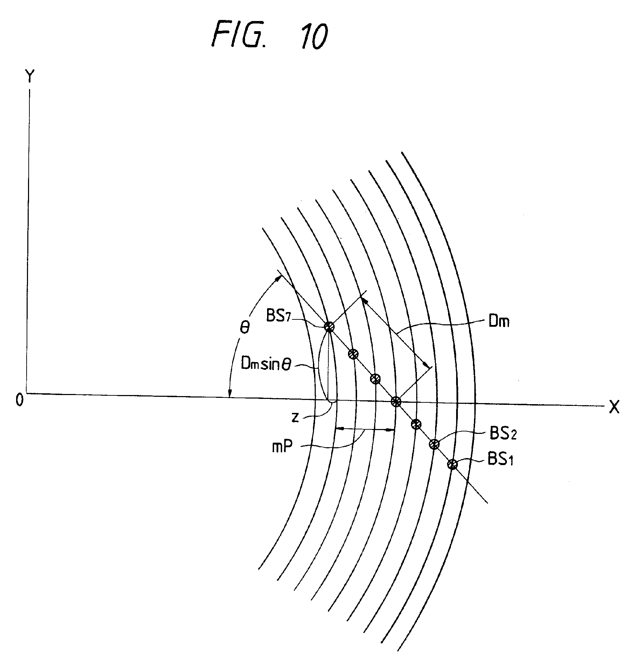

The intervals of these beam spots LB₁ through LB₇ are set as mentioned in the following part. First, as shown in Fig. 10, in case that the middle beam spot BS₄, among the beam spots BS₁ through BS₇ mentioned above, is arranged in such a way as to move along the line in the radial direction A of the optical disk 8 and also to be positioned on the track 17 of the optical disk 8, the beam spots are considered with being divided between inner side beam spots and outer side beam spots with respect to the beam spot BS₄.

First, the condition for the positioning of the beam spot BSₘ, which is in the m-th position on the inner side of the middle beam spot BS₄ mentioned above, on the track 17ₘ in the m-th position on the inner side of the track on which the middle beam spot BS₄ is positioned, is that the coordinates (x and y) of the beam spot BSₘ in the m-th position on the inner side should satisfy the equation for a circle expressive of the track 17ₘ in the m-th position of the inner side as counted from the track where the middle beam spot BS₄ is positioned. Thus,${\text{x² + y² = R}}_{\text{i}} \text{²}$

Where, Rᵢ represents the radius of the track 17ₘ in the m-th position on the inner side.

Now, as shown in Fig. 10, the difference between the radius Rᵢ of the track 17ₘ in the m-th position on the inner side and the X-coordinate of the beam spot BSₘ in the m-th position on the inner side is taken as Z while the distance from the middle beam spot BS₄ to the beam spot BSₘ in the m-th position on the inner side is taken as Dₘ, and then the equation (1) given above can be expressed as indicated in the following:${\text{(R}}_{\text{i}} {\text{- z)² + (D}}_{\text{m}} {\text{sin ϑ)² = R}}_{\text{i}} \text{²}$

Where, Z given above is expressed in the following equation as it is evident from Fig. 10.${\text{Z = D}}_{\text{m}} \text{cos ϑ - mp}$

Where, p expresses the pitch of the tracks 17 and 17, .... When this equation (3) is substituted into the equation (2) given above and arranged, the following equation is obtained:${\text{D}}_{\text{m}} {\text{² - 2D}}_{\text{m}} {\text{cos ϑ (mp + R}}_{\text{i}} {\text{) + (mp)² + 2mpR}}_{\text{i}} \text{= 0}$

Therefore, the distance Dₘ from the middle beam spot BS₄ to the beam spot BSₘ in the m-th position on the inner side is given by the following equation derived by solving the quadratic equation (4) given above:${\text{D}}_{\text{m}} {\text{= cos ϑ (mp + R}}_{\text{i}} \text{) +} \sqrt{{\text{R}}_{\text{i}} {\text{}}^{{\text{}}^{\text{2}}} {\text{- {sin [theta] (mp + R}}_{\text{i}} \text{)}²}}$

In the meanwhile, Dₘ, which expresses the distance from the middle beam spot BS₄ mentioned above to the beam spot BSₘ in the m-th position on the outer side of the middle beam spot BS₄, is given by the following equation in the same manner as described above:${\text{D}}_{\text{m}} {\text{= - R}}_{\text{i}} \text{cos ϑ +} \sqrt{{\text{(R}}_{\text{i}} {\text{cos ϑ)² + (mp)² + 2R}}_{\text{i}} \text{(mp)}}$

Therefore, the distance Dₘ from the middle beam spot BS₄ to the beam spot BSₘ in the m-th position either on the inner side or the outer side of the middle beam spot BS₄, with the equation (5) or the equation (6) given above being used therefor, and the intervals l₁ through l₆ of the individual beam spots BS₁ through BS₇ are obtained from this value as shown below. In this regard, the radius of curvature Rᵢ is set at 30 mm, the angle ϑ is set at 87 degrees, and the pitch p is set at 1.6 »m. As the result, the intervals l₁ through l₆ for the individual beam spots BS₁ through BS₇ are found to be as shown in the following:
l₁ = 28.8 »m
l₂ = 29.0 »m
l₃ = 29.8 »m
l₄ = 30.4 »m
l₅ = 31.0 »m
l₆ = 32.3 »m

Therefore, in case a plurality of beam spots BS₁ through BS₇ are arranged in a straight line with a slant at the angle ϑ in relation to the radial direction A of the optical disk 8, the intervals l₁ through l₆ of the beam spots BS₁ through BS₇ can be set at the value mentioned above in order to position all of the beam spots BS₁ through BS₇ on the tracks 17 and 17 ... on the optical disk 8.

Moreover, the laser diode 2, which emits the laser beam LB₁ through LB₇ mentioned above, has a plurality of laser diode elements 18₁ through 18₇ arranged at the intervals x₁ through x₆, which reflects consideration given to the image forming magnification of the image forming optical system 41, as shown in Fig. 1, so that the beam spots BS₁ through BS₇ are formed at the intervals l₁ through l₆ mentioned above.

In the construction described hereinabove, the multibeam head of the embodiment according to this invention performs, for example, the reproduction of information in the following manner. That is to say, in order to perform the reproduction of information, the laser diode 2 emits a plurality of laser beams LB₁ through LB₇, as shown in Fig. 2, and these laser beams LB₁ through LB₇ are directed onto the tracks 17 and 17 ... of the optical disk via the image forming optical system 41 along a straight line set with a slant at the angle ϑ in relation to the radial direction A of the optical disk 8 for their image formation as the beam spots BS₁ through BS₇.

The beam spots BS₁ through BS₇ formed on the optical disk 8 mentioned above have their intervals l₁ through l₆ increasing gradually, so that all the beam spots BS₁ through BS₇ are positioned on the tracks 17 and 17 ... of the optical disk 8.

Owing to this feature, it is possible in this embodiment to position all the laser beams LB₁ through LB₇ on the tracks 17 and 17 ... of the optical disk 8 at the same time and with accuracy even when it is attempted to attain a high transfer data rate in the reproduction of information from a plurality of tracks 17 and 17 of the optical disk 8 at the same time by the use of a plurality of the laser beams LB₁ through LB₇.

In this embodiment, there are a plurality of laser beams, and it is best to detect the servo signal and particularly the tracking error signal for performing the tracking servo through the middle spot, in view of the fact that the difference in the radius of curvature between the tracks 17 and 17 ... at the inner circumference and the outer circumference grows larger as given beams deviate from the axis of movement of the optical head, in case the number of the beams is an odd number.

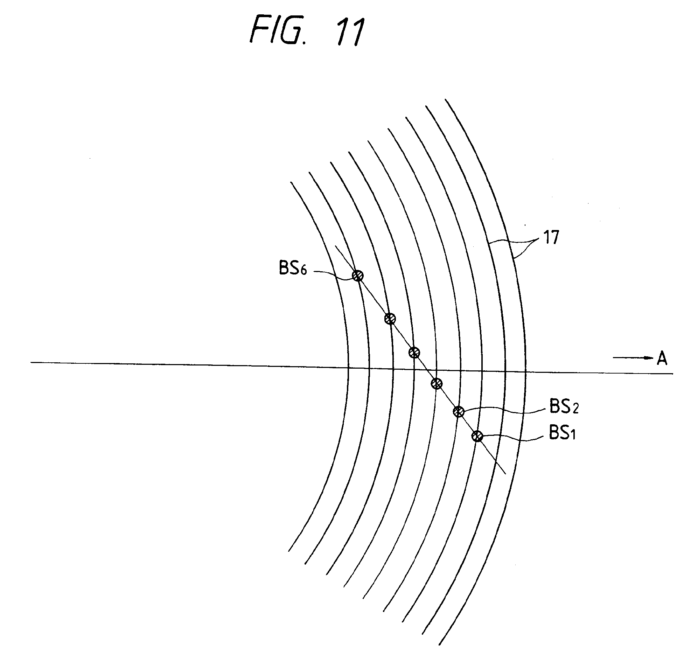

Fig. 11 shows another embodiment of this invention, and a description will now be made of this embodiment, with the same reference numbers being placed on the same parts as in the above described embodiment. In this embodiment, the system is set in such a way that the number of the laser beams is an even number, and, additionally, the spots of the individual laser beams are so arranged on the optical disk that the same numbers of the spots are positioned on both sides in relation to the axis of movement of the optical head.

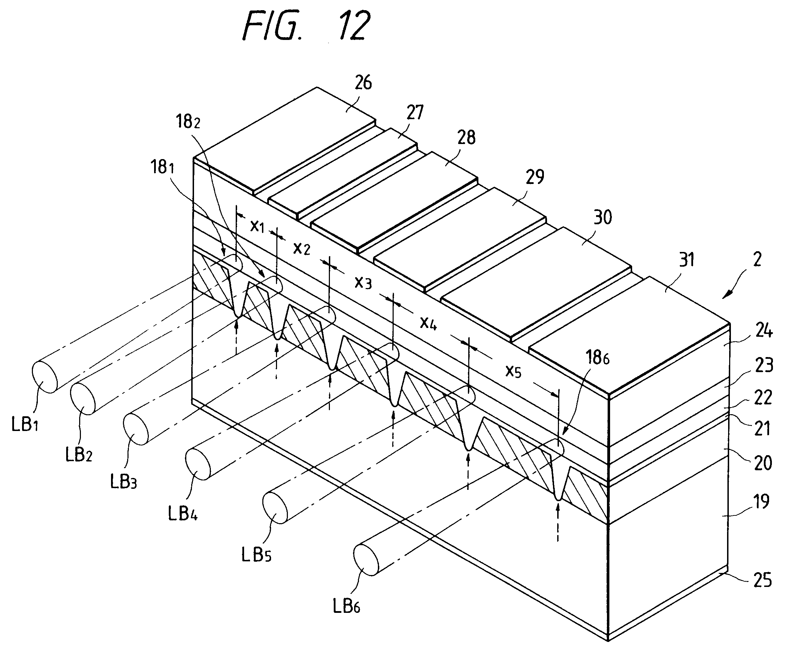

In specific terms, the laser diode 2 is provided with an even number of pieces of the laser diode elements 18₁ through 18₆ (six pieces in the Figure), as shown in Fig. 12, and the laser beams LB₁ through LB₆ emitted from the laser diode 2 are applied onto the optical disk 8 at the intervals l₁ through l₅ corresponding to the magnification of the image forming optical system 41 to form the beam spots BS₁ through BS₆, as shown in Fig. 11. In this case, the middle position of the two beam spots BS₃ and BS₄ in the middle is set to move in the radial direction A of the optical disk 8.

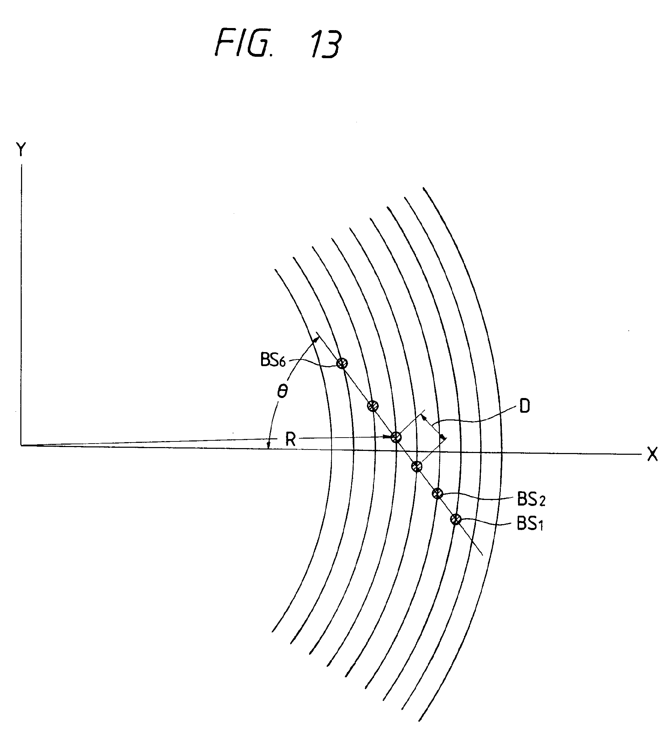

In this case, the intervals of the individual beam spots BS₁ through BS₆ are set as shown below on the basis of Fig. 13. That is to say, the distance Dₘ of the individual beam spots BS₁ through BS₆ is obtained by finding the crossing point of the equation for a circle expressing the tracks 17 and 17 ... and the equation for a straight line expressing the arrangement of the beam spots BS₁ through BS₆.

Accordingly, by the arithmetic operations performed as mentioned above, the distance Dₘ from the middle beam spot BS₄ to the beam spot BSₘ in the m-th position either on the inner side or the outer side from the middle beam spot BS₄ is calculated, and the intervals l₁ through l₅ of the individual beam spots BS₁ through BS₆ are obtained from this value as shown below. In this regard, the radius of curvature Rᵢ is set at 30 mm, the angle ϑ is set at 87 degrees, and the pitch p is set at 1.6 »m. As the result, the intervals l₁ through l₅ of the individual beam spots BS₁ through BS₆ are given as follows:
l₁ = 29.5 »m
l₂ = 30.0 »m
l₃ = 30.0 »m
l₄ = 30.6 »m
l₅ = 31.2 »m

It is thus possible to position all of the beam spots BS₁ through BS₆ on the tracks 17 and 17 ... of the optical disk 8, as shown in Fig. 11, by setting the intervals l₁ through l₅ for the beam spots BS₁ through BS₆ at the values mentioned above, when a plurality of beam spots BS₁ through BS₆ are arranged in a straight line with a slant at the angle ϑ in relation to the radial direction A of the optical disk 8.

Moreover, in case the number of the laser beams is thus an even number, there is no middle beam spot, unlike the case wherein the number of the laser beams is an odd number, but the tracking error signal for the performance of the tracking servo can be taken from either one of the two middle beams closest to the axis of movement of the optical head 40 or from the mean value of these.

The construction and working of the other parts of the system in this embodiment are the same as those of the example of embodiment described above, and their description is therefore omitted here.

This invention consists in the construction and working described above, and, since the laser diode according to this invention is provided with a plurality of light emitting elements arranged at varying intervals, the laser diode is capable of irradiating all of the laser beams emitted from the plurality of light emitting elements onto the tracks on the optical writing media at the same time.

Moreover, the multibeam optical head according to this invention is so constructed as to be provided with a laser diode in which a plurality of independently drivable light emitting elements are arranged in a straight line with their intervals varied and an image forming optical system which forms images in such a manner that a plurality of beams from the laser diode mentioned above are applied onto the optical writing media to form images arranged linearly at a prescribed angle in relation to the radial direction of the optical writing media and additionally that the intervals of the plural number of beam spots are varied. Hence, the multibeam optical head according to this invention is capable of directing all the plural number of laser beams emitted from the laser diode onto the tracks of the optical writing media via the image forming optical system to form the images at the same time even when it is designed to form the images arranged linearly at a prescribed angle to the radial direction of the optical writing media at the same time by applying the laser beams.

## Claims

1. An optical information system (1), comprising:
optical recording means (8) having a plurality of circular tracks (17) uniformely spaced one from another in a radial direction of said optical recording means (8) for recording information thereon; and
multibeam optical head means (40) optically coupled to said optical recording means (8) and movable along a movement axis in said radial direction, said multibeam optical head means (40) comprising:
laser diode means (2) having a plurality of lasing means (18₁, 18₂,..18₇) nonuniformely spaced one from another for generating a plurality of spaced laser beams (LB₁, LB₂,...LB₇), and
image forming optical means (41), coupled to said laser diode means (2), for directing each of said plurality of laser beams (LB₁, LB₂,...LB₇) as a beam spot (BS₁, BS₂,...BS₇) onto a respective one of said plurality of tracks (17) of the optical recording means (8) with a common amplification, so that said beam spots are arranged in a straight line set at a slant in relation to said radial direction,
characterized in that
said plurality of lasing means (18₁, 18₂,..18₇) is nonuniformely spaced one from another such that the spacing between adjacent ones of said beam spots (BS₁, BS₂,...BS₇) corresponds to the spacing between adjacent ones of said lasing (18₁, 18₂,..18₇) means.

2. The optical information system of claim 1, wherein the spacing between said adjacent lasing means (18₁, 18₂,..18₇) monotonically increases in the order of the position of said lasing means (18₁, 18₂,..18₇).

3. The optical information system of claim 1, wherein the number of said plurality of laser beams (LB₁, LB₂,...LB₇) is an even number.

4. The optical information system of claim 1, wherein the number of said plurality of laser beams (LB₁, LB₂,...LB₆) is an odd number.

5. The optical information system of claim 1, wherein said laser diode means (2) comprises an odd number of the lasing means (18₁, 18₂,..18₆).

6. The optical information system of claim 1, wherein said laser diode means (2) comprises an even number of the lasing means (18₁, 18₂,..18₇).

7. The optical information system of claim 1, said multibeam optical head means (40) further comprising:
means (5) for reflecting said plurality of laser beams (LB₁, LB₂,...LB₇) directed on the tracks (17);
means (16), coupled to said reflecting means (5), for detecting at least one reference laser beam (LB₄) of a plurality of reflected laser beams (LB₁, LB₂,...LB₇); and
means (51, 52, 53 ;54, 55, 56), coupled to said detecting means (16), for generating a signal corresponding to a detected reference laser beam (LB₄).

8. The optical information system of claim 1, wherein an equal number of said beam spots (BS₁, BS₂,...BS₇) are on each side of said movement axis.

9. The optical information system of claim 4, wherein one of said beam spots (BS₄) is on said movement axis.

10. The optical information system of claim 7, wherein said detecting means (16) include a plurality of photodetectors (16a,...16d).

11. The optical information system of claim 7, wherein said detecting means (16) include a pinhole (133) in the center of said detecting means.

12. The optical information system of claim 7, wherein said generating means (51, 52, 53 ;54, 55, 56) include a plurality of amplifiers.

## Patentansprüche

1. Optisches Informationssystem (1), das aufweist:
ein optisches Aufzeichnungsmittel (8) mit einer Vielzahl von kreisförmigen Spuren (17), die gleichmäßig voneinander in einer radialen Richtung des optischen Aufzeichnungsmittels (8) zum darauf Aufzeichnen von Information beabstandet sind; und
ein optisches Mehrstrahlkopfmittel (40), das optisch mit dem optischen Aufzeichnungsmittel (8) gekoppelt und entlang einer Bewegungsachse in der radialen Richtung beweglich ist, wobei das optische Mehrstrahlkopfmittel (40) aufweist:
ein Laserdiodenmittel (2), das eine Vielzahl von ungleichmäßig voneinander beabstandeten Lasermitteln (18₁, 18₂,...18₇) aufweist, um eine Vielzahl von beabstandeten Laserstrahlen (LB₁, LB₂, ...LB₇) zu erzeugen, und
ein bilderzeugendes optisches Mittel (41), das mit dem Laserdiodenmittel (2) verbunden ist, um jeden aus der Vielzahl der Laserstrahlen (LB₁, LB₂, ...LB₇) als einen Strahlpunkt (BS₁, BS₂, ...BS₇) auf eine entsprechende Spur aus der Vielzahl der Spuren (17) des optischen Aufzeichnungsmittels (8) mit einer gemeinsamen Verstärkung zu lenken, so daß die Strahlpunkte in einer geraden Linie ausgerichtet sind, die unter einem Winkel bezüglich der radialen Richtung eingestellt ist,
dadurch gekennzeichnet, daß
die Vielzahl der Lasermittel (18₁, 18₂, ...18₇) ungleichmäßig voneinander beabstandet ist, so daß der Abstand zwischen benachbarten Strahlpunkten (BS₁, BS₂, ...BS₇) dem Abstand zwischen benachbarten Lasermitteln (18₁, 18₂, ..18₇) entspricht.

2. Optisches Informationssystem nach Anspruch 1, worin der Abstand zwischen benachbarten Lasermitteln (18₁, 18₂, ...18₇) monoton in der Reihenfolge der Position der Lasermittel (18₁, 18₂, ...18₇) zunimmt.

3. Optisches Informationssystem nach Anspruch 1, worin die Anzahl der Vielzahl der Laserstrahlen (LB₁, LB₂, ...LB₇) eine gerade Zahl ist.

4. Optisches Informationssystem nach Anspruch 1, worin die Anzahl der Vielzahl der Laserstrahlen (LB₁, LB₂, ...LB₆) eine ungerade Zahl ist.

5. Optisches Informationssystem nach Anspruch 1, worin das Laserdiodenmittel (2) eine ungerade Anzahl der Lasermittel (18₁, 18₂, ...18₆) umfaßt.

6. Optisches Informationssystem nach Anspruch 1, worin das Laserdiodenmittel (2) eine gerade Anzahl der Lasermittel (18₁, 18₂, ...18₇) umfaßt.

7. Optisches Informationssystem nach Anspruch 1, worin das optische Mehrstrahlkopfmittel (40) weiterhin aufweist:
ein Mittel (5) zum Reflektieren der auf die Spuren (17) gerichteten Vielzahl der Laserstrahlen (LB₁, LB₂, ...LB₇);
ein mit dem reflektierenden Mittel (5) verbundenes Mittel (16) zum Detektieren von mindestens einem Referenzlaserstrahl (LB₄) aus einer Vielzahl von reflektierten Laserstrahlen (LB₁, LB₂, ...LB₇); und
ein mit dem detektierenden Mittel (16) verbundenes Mittel (51, 52, 53; 54, 55, 56) zum Erzeugen eines Signals, das einem detektierten Referenzlaserstrahl (LB₄) entspricht.

8. Optisches Informationssystem nach Anspruch 1, worin eine gleiche Anzahl der Strahlpunkte (BS₁, BS₂, ...BS₇) sich auf jeder Seite der Bewegungsachse befindet.

9. Optisches Informationssystem nach Anspruch 4, worin einer der Strahlpunkte (BS₄) sich auf der Bewegungsachse befindet.

10. Optisches Informationssystem nach Anspruch 7, worin das Detektionsmittel (16) eine Vielzahl von Fotodetektoren (16a, ...16d) umfaßt.

11. Optisches Informationssystem nach Anspruch 7, worin das Detektionsmittel (16) ein kleines Loch (133) in dem Zentrum des detektierenden Mittels aufweist.

12. Optisches Informationssystem nach Anspruch 7, worin das Erzeugungsmittel (51, 52, 53; 54, 55, 56) eine Vielzahl von Verstärkern umfaßt.

## Revendications

1. Système d'information optique (1), comprenant :
un moyen d'enregistrement optique (8) possédant une pluralité de pistes circulaires (17) uniformément séparées les unes des autres suivant la direction radiale dudit moyen d'enregistrement optique (8) et servant à enregister des informations ; et
un moyen (40) tête optique multifaisceau qui est optiquement couplé audit moyen d'enregistrement optique (8) et peut être déplacé le long d'un axe de déplacement suivant ladite direction radiale, ledit moyen tête optique multifaisceau (40) comprenant :
un moyen diodes laser (2) possédant une pluralité de moyens d'émission laser (18₁, 18₂, ... 18₇) séparés les uns des autres de façon non uniforme de façon à produire une pluralité de faisceaux laser séparés (LB₁, LB₂, ... LB₇), et
un moyen optique (41) de formation d'image, couplé audit moyen diodes laser (2) afin de diriger chacun desdits faisceaux laser (LB₁, LB₂, ... LB₇) sous la forme d'une tache de faisceau (BS₁, BS₂, ... BS₇) sur l'une respective desdites pistes (17) du moyen d'enregistrement optique (8) avec une amplification commune, de façon que lesdites taches de faisceaux soient disposées sur une ligne droite fixée suivant une inclinaison par rapport à ladite direction radiale,
caractérisé en ce que :
lesdits moyens d'émission laser (18₁, 18₂,... 18₇) sont séparés les uns des autres de façon non uniforme de façon que l'écartement entre celles desdites taches de faisceau (BS₁, BS₂, ... BS₇) qui sont adjacentes corresponde à l'écartement entre ceux desdits moyens d'émission laser (18₁, 18₂, ... 18₇) qui sont adjacents.

2. Système d'information optique selon la revendication 1, où l'écartement entre lesdits moyens d'émission laser adjacents (18₁, 18₂, ... 18₇) augmente de façon monotone dans l'ordre de la position desdits moyens d'émission laser (18₁, 18₂, ... 18₇).

3. Système d'information optique selon la revendication 1, où le nombre desdits faisceaux laser (LB₁, LB₂, ... LB₇) est un nombre pair.

4. Système d'information optique selon la revendication 1, où le nombre desdits faisceaux laser (LB₁, LB₂, ... LB₆) est un nombre impair.

5. Système d'information optique selon la revendication 1, où ledit moyen diodes laser (2) comprend un nombre impair des moyens d'émission laser (18₁, 18₂, ... 18₆).

6. Système d'information optique selon la revendication 1, où ledit moyen diodes laser (2) comprend un nombre pair des moyens d'émission laser (18₁, 18₂, ... 18₇).

7. Système d'information optique selon la revendication 1, ledit moyen tête optique multifaisceau (40) comprenant en outre :
un moyen (5) servant à réfléchir lesdits faisceaux laser (LB₁, LB₂, ... LB₇) dirigés sur les pistes (17) ;
un moyen (16), couplé audit moyen réfléchissant (5), afin de détecter au moins un faisceau laser de référence (LB₄) d'une pluralité de faisceaux laser réfléchis (LB₁, LB₂,... LB₇) ; et
un moyen (51, 52, 53 ; 54, 55, 56), couplé audit moyen de détection (16), afin de produire un signal correspondant à un faisceau laser de référence détecté (LB₄).

8. Système d'information optique selon la revendication 1, où un nombre égal desdites taches de faisceau (BS₁, BS₂, ... BS₇) se trouve sur chaque côté dudit axe de déplacement.

9. Système d'information optique selon la revendication 4, où une desdites taches de faisceau (BS₄) se trouve sur ledit axe de déplacement.

10. Système d'information optique selon la revendication 7, où ledit moyen de détection (16) comporte une pluralité de photodétecteurs (16a, ... 16d).

11. Système d'information optique selon la revendication 7, où ledit moyen de détection (16) comporte un trou d'épingle (133) formé au centre dudit moyen de détection.

12. Système d'information optique selon la revendication 7, où ledit moyen de production (51, 52, 53 ; 54, 55, 56) comporte une pluralité d'amplificateurs.
